(19) **Europäisches Patentamt**
**European Patent Office**
**Office européen des brevets**

(11) **EP 2 698 114 B1**

(12) **EUROPEAN PATENT SPECIFICATION**

(45) Date of publication and mention
of the grant of the patent:
**16.08.2017 Bulletin 2017/33**

(21) Application number: **12772003.5**

(22) Date of filing: **05.04.2012**

(51) Int Cl.:
*G01S 7/52* (2006.01)     *A61B 8/00* (2006.01)
*A61B 8/13* (2006.01)     *G06T 19/20* (2011.01)
*A61B 8/08* (2006.01)     *G06T 7/73* (2017.01)
*G06T 7/11* (2017.01)     *G06T 7/60* (2017.01)
*G06T 7/155* (2017.01)

(86) International application number:
**PCT/JP2012/059346**

(87) International publication number:
**WO 2012/141068 (18.10.2012 Gazette 2012/42)**

(54) **ULTRASOUND DIAGNOSTIC DEVICE**

ULTRASCHALLDIAGNOSEVORRICHTUNG

DISPOSITIF DE DIAGNOSTIC ÉCHOGRAPHIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **14.04.2011 JP 2011089927**

(43) Date of publication of application:
**19.02.2014 Bulletin 2014/08**

(73) Proprietor: **Hitachi, Ltd.**
**Chiyoda-ku,**
**Tokyo 100-8280 (JP)**

(72) Inventors:
• **NAGASE, Yuko**
**Mitaka-shi**
**Tokyo 181-8622 (JP)**

• **MATSUSHITA, Noriyoshi**
**Mitaka-shi**
**Tokyo 181-8622 (JP)**

(74) Representative: **Heim, Florian Andreas et al**
**Weber & Heim**
**Patentanwälte**
**Partnerschaftsgesellschaft mbB**
**Irmgardstraße 3**
**81479 München (DE)**

(56) References cited:
JP-A- 2002 011 000    JP-A- 2002 330 951
JP-A- 2004 313 651    JP-A- 2006 146 393
JP-A- 2006 341 092    JP-A- 2008 521 461
JP-A- 2009 072 400    JP-A- 2009 165 615
US-A1- 2006 280 351    US-A1- 2006 285 734
US-A1- 2008 267 499    US-A1- 2009 082 668
US-A1- 2010 195 881

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

TECHNICAL FIELD

The present invention relates to an ultrasound diagnostic apparatus, and in particular to a technique for forming a display image of a diagnosis target.

BACKGROUND ART

Techniques for displaying an ultrasound image of a tissue or the like in a display image suited for diagnosis are known from the related art, and various display images exist as the display image for these techniques, corresponding to the type of the tissue or the like and the contents of the diagnosis. As the tissue or the like, a plurality of follicles in a living body are in some cases a target of ultrasound diagnosis.

Each follicle is in many cases observed in a shape approximately close to an ellipse, and, for example, a major axis of each follicle along a longitudinal direction thereof and minor axes orthogonal to the major axis are used as measurements in diagnosis of each follicle. Because of this, for example, many users desire a cutting-plane image including the major axis and the minor axes of the follicle when the ultrasound image data is three-dimensionally obtained and the cutting-plane image of each follicle is displayed.

However, in the process of setting the position of the cutting plane for the follicle to include the major axis and the minor axes of the follicle, if the setting is to be manually performed by the user, the user is forced to execute complicated operation. In particular, in a living body, a large number of follicles exist close to each other at high density, and the manual setting of the cutting plane for each of the large number of follicles requires a great amount of work.

In such a circumstance, the present inventors have researched and developed techniques for forming a display image suitable for diagnosis for a diagnosis target such as, for example, the follicle. In the formation of the display image, it is desirable to refer to the form of the diagnosis target; that is, the size and shape of the diagnosis target. An example of a feature quantity showing the form of the diagnosis target is the major axis identified along a longitudinal direction of the diagnosis target (refer to Patent Document 1).

[Related Art References]

[Patent Document]

[Patent Document 1] Japanese Patent No. 3802508

Further documents relating to image processing of data which was acquired by ultrasonic waves are known from US 2009/0082668 A1, US 2006/0280351 A1, US 2008/0267499 A1, US 2010/0195881 A1 or/and US 2006/0285734 A1.

DISCLOSURE OF INVENTION

[Technical Problem]

The present invention was conceived in the process of the above-described research and development, and an advantage thereof is realization of a display image of a diagnosis target according to the form of the diagnosis target.

[Solution to Problem]

According to one aspect of the present invention, there is provided an ultrasound diagnostic apparatus comprising a probe which transmits and receives ultrasound to and from a diagnostic region; a transmitting and receiving unit which controls the probe to obtain a reception signal from the diagnostic region; a target identifying unit which identifies image data of a diagnosis target in image data of the diagnostic region formed based on the reception signal; a coordinate system setting unit which sets, based on the image data of the diagnosis target, a diagnostic coordinate system based on a form of the diagnosis target; a coordinate system matching unit which matches with each other a display coordinate system forming a basis of a display image and the diagnostic coordinate system, to place the image data of the diagnosis target in the display coordinate system; and a display image forming unit which forms a display image of the diagnosis target based on the image data of the diagnosis target placed in the display coordinate system.

In the above-described configuration, the image data of the diagnostic region may be formed, for example, with a plurality of echo data which are two-dimensionally arranged or a plurality of voxel data which are three-dimensionally arranged. When the image data of the diagnostic region are two-dimensional data, the diagnostic coordinate system and the display coordinate system are desirably two-dimensional coordinates. When the image data of the diagnostic region are three-dimensional data, the diagnostic coordinate system and the display coordinate system are desirably three-dimensional coordinates or two-dimensional coordinates. In addition, although the diagnostic coordinate system and the display coordinate system are desirably orthogonal coordinate systems, there may be used coordinate systems other than the orthogonal coordinate system, such as coordinate systems suited for the form of scanning of the ultrasound probe.

In the above-described configuration, the display coordinate system and the diagnostic coordinate system are matched with each other. For example, one coordinate system is placed in the other coordinate system such that a certain matching condition is satisfied. The matching condition is, for example, a relative placement relationship between a coordinate axis and a coordinate plane defining one coordinate system, and a coordinate axis and a coordinate plane defining the other coordinate system, or the like. As an example, there exists a form in which one coordinate axis of the display coordinate system and one coordinate axis of the diagnostic coordinate system are overlapped with each other. Alternatively, one coordinate axis of the display coordinate system and one coordinate axis of the diagnostic coordinate system may be matched in a manner to intersect each other with a certain intersection angle.

With the above-described configuration, because the diagnostic coordinate system based on the form of the diagnosis target and the display coordinate system are matched with each other when the image data of the diagnosis target are to be placed in the display coordinate system, placement of the image data corresponding to the form of the diagnosis target is realized, and a display image of the diagnosis target according to the form of the diagnosis target can be formed.

According to another aspect of the present invention, there is provided an ultrasound image processor comprising a target identifying unit which identifies image data of a diagnosis target in ultrasound image data; a coordinate system setting unit which sets, based on the image data of the diagnosis target, a diagnostic coordinate system based on a form of the diagnosis target; a coordinate system matching unit which matches with each other the display coordinate system forming a basis of a display image and the diagnostic coordinate system, to place the image data of the diagnosis target in the display coordinate system; and a display image forming unit which forms a display image of the diagnosis target based on the image data of the diagnosis target placed in the display coordinate system.

According to another aspect of the present invention, for example, a program which realizes the functions of the target identifying unit, the coordinate system setting unit, and the coordinate system matching unit described above may be used to cause a computer to realize these functions so that the computer functions as the ultrasound image preprocessor described above.

[Advantageous Effects of Invention]

According to various aspects of the present invention, a display image of a diagnosis target according to the form of the diagnosis target can be formed.

BRIEF DESCRIPTION OF DRAWINGS

FIG. 1 is a diagram showing an overall structure of an ultrasound diagnostic apparatus preferable for practicing the present invention.
FIG. 2 is a diagram for explaining a process performed in a target identifying unit.
FIG. 3 is a diagram for explaining scanning of a filter in a three-dimensional data space.
FIG. 4 is a diagram for explaining a filter process in a dilation process.
FIG. 5 is a diagram for explaining a major axis and two minor axes of a follicle.
FIG. 6 is a diagram showing a diagnostic coordinate system based on a follicle.
FIG. 7 is a diagram for explaining matching of a display coordinate system and a diagnostic coordinate system.
FIG. 8 is a diagram for explaining a cross section based on a display coordinate system.
FIG. 9 is a diagram showing a concrete example of a display image.

BEST MODE FOR CARRYING OUT THE INVENTION

FIG . 1 is a diagram showing an overall structure of an ultrasound diagnostic apparatus prefer able in practicing the present invention. A probe 10 is an ultrasound probe which transmits and receives ultrasound to and from a region including a diagnosis target. The probe 10 comprises a plurality of transducer elements which transmit and receive ultrasound. The plurality of transducer elements are transmission-controlled by a transmitting and receiving unit 12, to form a transmission beam. The plurality of transducer elements also receive ultrasound obtained from the region including a diagnosis target; a signal thus obtained is output to the transmitting and receiving unit 12; the transmitting and receiving unit 12 forms a reception beam; and echo data are collected along the reception beam.

As the probe 10, a three-dimensional probe which scans the ultrasound beam (transmission beam and reception beam) in a three-dimensional space and three-dimensionally collects the echo data is preferable. For example, a scanning plane electrically formed by a plurality of transducer elements which are arranged one-dimensionally (1-D array transducer) may be mechanically moved to three-dimensionally scan the ultrasound beam. Alternatively, a plurality of transducer elements arranged two-dimensionally (2-D array transducer) may be electrically controlled to three-dimensionally scan the ultrasound beam. Alternatively,there may be employed a two-dimensional ultrasound probe which scans the ultrasound beam within a tomographic plane.

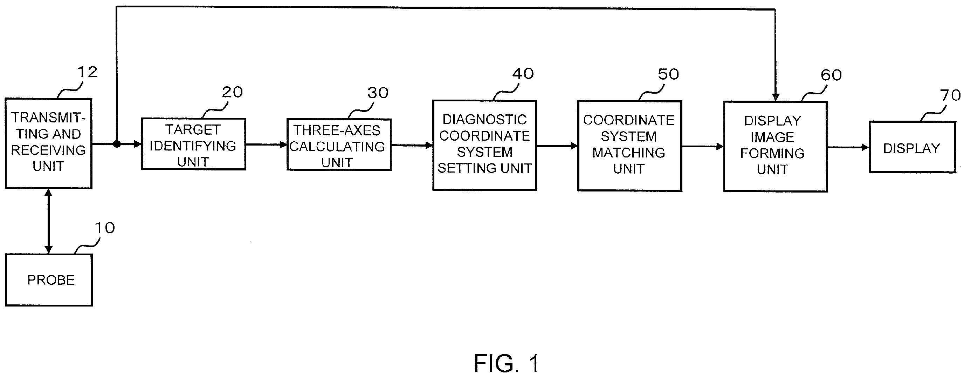

When the ultrasound beam is scanned in the three-dimensional space and echo data are collected, echo data (voxel data) for a plurality of voxels forming the three-dimensional data space corresponding to the three-dimensional space are stored in a memory or the like (not shown). For the plurality of voxels forming the three-dimensional data space, various processes are executed by a target identifying unit 20 and the subsequent units. These processes will now be described. For a portion (structure) shown in FIG. 1, the reference numerals of FIG. 1 are used in the following description.

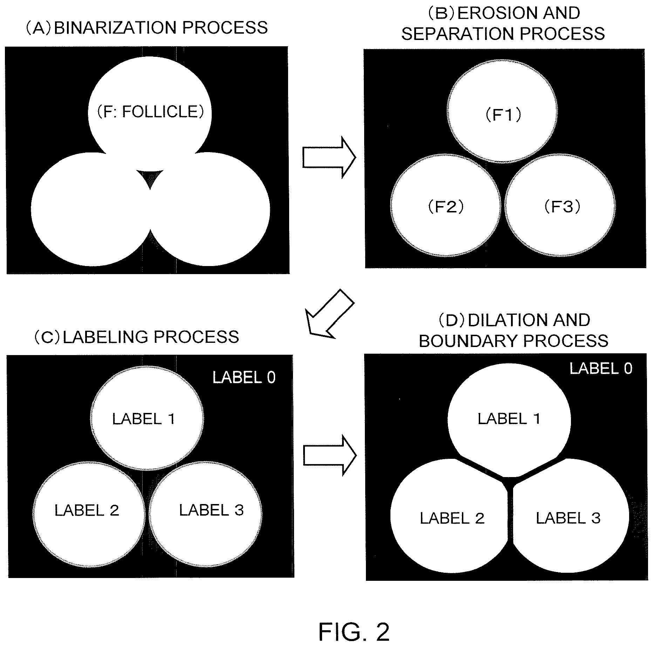

FIG. 2 is a diagram for explaining a process performed in the target identifying unit 20. FIG. 2(A) shows a binarization process. The target identifying unit 20 applies a binarization process on the plurality of voxels forming the three-dimensional data space, to form image data after the binarization process shown in FIG. 2(A). As the diagnosis target in the present embodiment, a follicle in a living body is preferable. The target identifying unit 20 compares the voxel value of each voxel (magnitude of echo data) with a threshold value for binarization, to distinguish voxels that correspond to the follicle F and voxels that do not. Then, for example, a voxel value of a voxel corresponding to the follicle F is set to "1" and a voxel value of the other voxels is set to "0." In FIG. 2(A), a group of voxels corresponding to the follicle F is shown by a white color and a group of the other voxels corresponding to the background is shown by a black color.

In a living body, the plurality of follicles exist at high density, very close to each other. Therefore, in the ultrasound image, as shown in FIG. 2(A), an image of the plurality of follicles F is formed such that the follicles F are connected to each other, and it is difficult to individually check the size, shape, etc., of each follicle F. In consideration of this, in the present embodiment, the plurality of follicles F are separated into individual follicles by various processes to be described below. In FIG. 2, although each set of image data is two-dimensionally drawn, the processes are three-dimensionally executed in the three-dimensional data space.

FIG. 2(B) shows an erosion and separation process. The target identifying unit 20 applies an erosion process on the plurality of follicles F in the voxel data forming the three-dimensional data space and to which the binarization process is applied; that is, in the binarized image data shown in FIG. 2(A), the plurality of follicles F are separated into follicles F1 - F3, as shown in FIG. 2(B). The target identifying unit 20 repeatedly executes the erosion process to stepwise erode the follicle F n times (where n is a natural number). For the erosion process of each step, a filter for the erosion process is used, and the filter is scanned over the entire region of the three-dimensional data space.

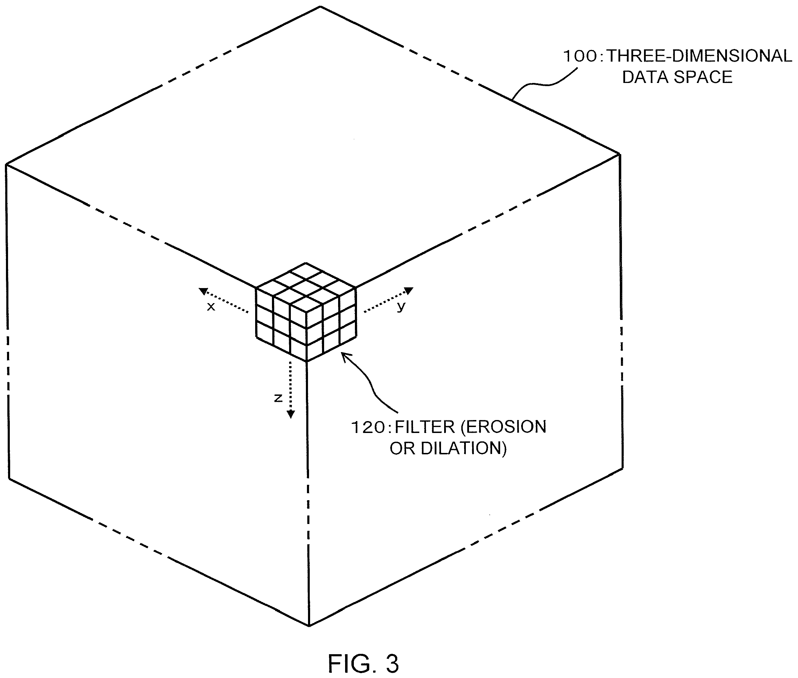

FIG. 3 is a diagram for explaining scanning of a filter 120 in three-dimensional data space 100. In FIG. 3, the three-dimensional data space 100 is shown with an xyz orthogonal coordinate system. In addition, the filter 120 has a three-dimensional structure with lengths in the x-axis direction, the y-axis direction, and z-axis direction each corresponding to three voxels, and, consequently, with a volume corresponding to a total of 27 voxels. A voxel positioned at the center of the filter 120 is a voxel of interest, and 26 voxels surrounding the voxel of interest are peripheral voxels. The filter 120 is scanned over the entire region of the three-dimensional data space 100 by being moved in the x-axis direction, the y-axis direction, and the z-axis direction, so that each of the voxels in the three-dimensional data space 100 is set as the voxel of interest.

In the erosion and separation process, at each scan position, if there is at least one voxel with the voxel value of "0" among the 26 peripheral voxels in the filter 120, the voxel value of the voxel of interest positioned at the center of the filter 120 is set as "0." For example, when the voxel of interest has a voxel value of "1" (follicle), and at least one of the peripheral voxels has a voxel value of "0" (background), the voxel value of the voxel of interest is converted to "0" (background). By the filter 120 being scanned once over the entire region of the three-dimensional data space 100 and the filtering process being executed for each scan position, the erosion process of one step is completed. The conversion of the voxel value with regard to the voxel of interest is executed after the filter 120 is scanned once over the entire region of the three-dimensional data space 100. In other words, the conversion of voxel value is not executed in the middle of scanning of the filter 120, and the filter process is executed at any scan position based on the voxel value before the conversion.

When the erosion process of one step is completed as described above and the voxel value is converted based on the result of the erosion process, an erosion process of a second step is executed on the three-dimensional data space 100 formed of the converted voxel values. In the erosion process of second step also, the same filter process as the erosion process of the first step is executed. Specifically, in each scan position, if there is at least one voxel with a voxel value of "0" among the 26 peripheral voxels in the filter 120, the voxel value of the voxel of interest positioned at the center of the filter 120 is converted to "0." The conversion of the voxel value is executed after the filter 120 is once scanned over the entire region of the three-dimensional data space 100.

The target identifying unit 20 repeatedly executes the stepwise erosion process n times (where n is a natural number). The number of repetitions n is suitably determined according to the size of each voxel, the size of the filter, etc., and is set, for example, to be about 10 or less. Alternatively, there may be employed a configuration in which the user can adjust the number n.

In the case of two-dimensional configuration, in place of the filter 120 shown in FIG. 3, a two-dimensional filter having the length and width corresponding to 3 voxels, and consequently, an area of a total of 9 voxels, may be used, a voxel positioned at the center may be set as the voxel of interest, and the 8 voxels surrounding the voxel of interest may be set as the peripheral voxel.

Referring again to FIG. 2, when the data are separated as a result of the erosion process into a plurality of follicles F1 - F3 as shown in FIG. 2(B), the target identifying unit 20 applies a labeling process in the voxel data forming the three-dimensional data space and to which the erosion process is applied; that is, in the image data after the erosion process shown in FIG. 2(B), different labels are assigned to the plurality of follicles F1 - F3. As the labeling process, known methods may be employed. For example, a block of a plurality of voxels having the same voxel value in the three-dimensional data space is detected, and a label number is assigned for each block. For example, as shown in FIG. 2(C), a label of 0 is assigned to the background portion which is a block with the voxel value of "0, " and labels of 1 - 3 are assigned to the follicles F1 - F3, respectively, which are blocks with the voxel value of "1."

After the labeling process is applied, the target identifying unit 20 applies a dilation process on each of a plurality of follicles in the voxel data forming the three-dimensional data space and to which the labeling process is applied; that is, the image data after the labeling process shown in FIG. 2(C). In a dilation portion obtained from each follicle in the dilation process, the label of the follicle is assigned, and the sizes of the plurality of follicles are restored while a boundary is formed at an overlap portion of the dilation portions (dilated follicle) which overlap each other due to the dilation process. With this process, as shown in FIG. 2(D), while the boundary (background pixel) is formed between the follicles corresponding to labels different from each other, the sizes of the follicles are restored to the sizes before the erosion process (immediately after the binarization process).

The target identifying unit 20 repeatedly executes the dilation process to stepwise dilate the follicle F n times (where n is the same number as the number of erosion processes). In the dilation process at each step, a filter for the dilation process is used, and the filter is scanned over the entire region in the three-dimensional data space. In the dilation process also, the three-dimensional filter 120 corresponding to a total of 27 voxels shown in FIG. 3 is used, a voxel positioned at the center of the filter 120 is set as the voxel of interest, and the 26 voxels surrounding the voxel of interest are set as the peripheral voxels. The filter 120 is moved in the x-axis direction, the y-axis direction, and the z-axis direction and scanned over the entire region of the three-dimensional data space 100, so that each of the voxels in the three-dimensional data space 100 is set as the voxel of interest. However, the filter process in the dilation process differs from that in the erosion process.

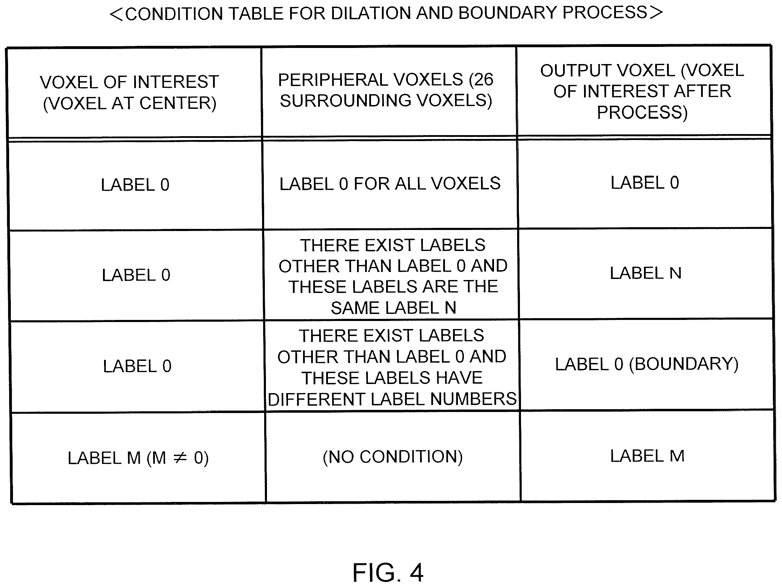

FIG. 4 is a diagram for explaining a filter process in the dilation process. FIG. 4 shows a condition table related to the conversion of the voxel value in the process to dilate while forming the boundary (dilation and boundary process). In the dilation and boundary process, reference is made to the label value of each voxel.

In the case where the voxel of interest positioned at the center of the filter 120 (FIG. 3) has a label of 0 (background), if all of 26 peripheral voxels have the label of 0 (background), the voxel of interest is set to a label of 0. In the case where the voxel of interest has the label of 0 (background), if there is a label (follicle) other than the label of 0 among the 26 peripheral voxels and all of the labels are the same label of N (same follicle), the voxel of interest is converted to the label of N. In other words, the follicle of the label of N is dilated.

In the case when the voxel of interest has a label of 0 (background), if there are labels (follicle) other than the label of 0 among the 26 peripheral voxels and the labels include different label numbers (follicles different from each other), the voxel of interest is set to the label of 0. In other words, the voxel of interest is maintained at the label of 0, and becomes a boundary between follicles which differ from each other.

On the other hand, when the voxel of interest positioned at the center of the filter 120 has a label of M (follicle), the voxel of interest is maintained with the label of M regardless of the status of the peripheral voxels.

When the filter 120 shown in FIG. 3 is scanned once over the entire region of the three-dimensional data space 100 and the filter process is applied at each scan position according to the condition shown in FIG. 4, a dilation and boundary process of one step is completed. The conversion of the label value of the voxel of interest is executed after the filter 120 is scanned once over the entire region of the three-dimensional data space 100. In other words, in the middle of scanning of the filter 120, the conversion of the label value is not executed, and the filter process is executed for the label value before the conversion for all scan positions.

When the dilation and boundary process of one step is completed in this manner and the label value is converted based on the result, the dilation and boundary process of a second step is executed on the three-dimensional data space 100 formed of the converted label values. In the dilation and boundary process of the second step also, the filter process identical to that of the first step is executed. Specifically, at each scan position, the filter process is executed according to the condition shown in FIG. 4, and, after the filter 120 is scanned once over the entire region of the three-dimensional data space 100, the label value is converted.

The target identifying unit 20 repeatedly executes the stepwise dilation and boundary process n times. The number of repetitions n is desirably identical to the number of repetitions n of the erosion process. In this manner, as shown in FIG. 2(D), while a boundary (background pixel) is formed between follicles corresponding to labels different from each other, the size of each follicle is restored to the size before the erosion process.

In the dilation and boundary process also, when a two-dimensional configuration is employed, in place of the filter 120 shown in FIG. 3, a two-dimensional filter corresponding to a length and a width of 3 voxels and a total of 9 voxels may be used, a voxel positioned at the center may be set as the voxel of interest, and the 8 voxels surrounding the voxel of interest may be set as the peripheral voxels.

In the present embodiment, as shown in FIG. 2, the plurality of follicles which exist at high density and close to each other are separated from each other and identified. In addition, as shown in FIG. 2(D), when an individual label is correlated to each follicle, corresponding follicles can be identified with the labels, and, for each label, calculation or the like of the measurement values related to the size and shape of each follicle corresponding to the label can be enabled. For example, a volume, a length of the major axis, a length of a minor axis, or the like of each follicle corresponding to each label may be calculated for each label.

In the process of identifying each follicle, for example, the user can designate a desired label to identify the follicle corresponding to the label. In addition, because the follicles are separated, on an image displaying the plurality of follicles, the user may designate a desired follicle by operating a display form such as a cursor, so that only an image of the follicle thus designated is displayed.

In the present embodiment, when the follicle designated by the user is displayed, a display image corresponding to the form of the follicle is formed. As a feature quantity related to the form of the follicle, a three-axes calculating unit 30 shown in FIG. 1 identifies a major axis and two minor axes of the follicle.

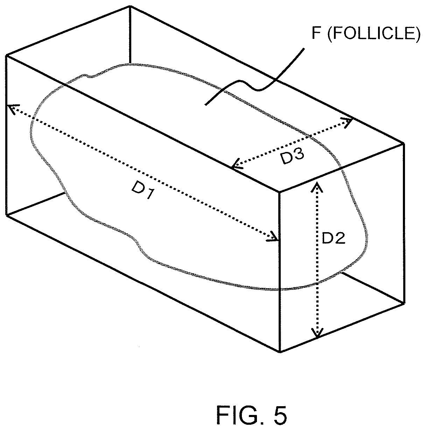

FIG. 5 is a diagram for explaining a major axis and two minor axes of the follicle. In the present embodiment, for the identified follicle F, as shown in FIG. 5, a minimum rectangular parallelepiped circumscribing the follicle F is considered, and lengths of the sides of the rectangular parallelepiped are set as the three axial lengths of the follicle F. For example, the longest side D1 shown in FIG. 5 is set as the major axis of the follicle F, and the sides D2 and D3 orthogonal to the side D1 are set as two minor axes of the follicle F.

Referring again to FIG. 1, the three-axes calculating unit 30 uses a method of primary component analysis in order to identify the three axes of the follicle. In the known method of primary component analysis, a direction which most represents the variation of the data; that is, a direction having the maximum variance of the data, is set as a first primary component. In the present embodiment, in the primary component analysis, for example, the following known covariance matrix is used.

In order to obtain a covariance matrix, an average position m is calculated by Equation 1. In Equation 1, $P_i$ represents a coordinate value in the three-dimensional data space (refer to FIG. 3) for an ith pixel (voxel) forming the follicle, and the average position (position of the center of gravity) m is calculated using the coordinate values of all pixels (voxels) of i = 1 - N forming the identified follicle.

[Equation 1]

$$m = \frac{1}{N}\sum_{i=1}^{N} P_i \qquad , \quad P_i = \langle x_i, y_i, z_i \rangle$$

Using the average position m of Equation 1, a covariance matrix C shown in Equation 2 is calculated. The covariance matrix C shown in Equation 2 is a 3 x 3 matrix, and is a symmetric matrix having 6 independent components shown in Equation 3.

[Equation 2]

$$m = \frac{1}{N}\sum_{i=1}^{N} P_i \qquad , \qquad P_i = \left\langle x_i, y_i, z_i \right\rangle$$

[Equation 3]

$$C_{11} = \frac{1}{N}\sum_{i=1}^{N}(x_i - m_x)^2 \qquad\qquad C_{12} = C_{21} = \frac{1}{N}\sum_{i=1}^{N}(x_i - m_x)(y_i - m_y)$$

$$C_{22} = \frac{1}{N}\sum_{i=1}^{N}(y_i - m_y)^2 \qquad\qquad C_{13} = C_{31} = \frac{1}{N}\sum_{i=1}^{N}(x_i - m_x)(z_i - m_z)$$

$$C_{33} = \frac{1}{N}\sum_{i=1}^{N}(z_i - m_z)^2 \qquad\qquad C_{23} = C_{32} = \frac{1}{N}\sum_{i=1}^{N}(y_i - m_y)(z_i - m_z)$$

In the primary component analysis using the covariance matrix C, eigenvectors of the covariance matrix C obtained by Equations 2 and 3 are calculated, and an eigenvector corresponding to a maximum eigenvalue is set as the first primary component. In the present embodiment, a direction of the first primary component obtained using the covariance matrix C is set as the major axis of the follicle. With this process, the major axis passing through the center of gravity of the follicle and along the longitudinal direction of the follicle is identified. In addition, directions of a second primary component and a third primary component obtained using the covariance matrix C are set as the two minor axes of the follicle. For example, a direction of the second primary component is set as a first minor axis and a direction of the third primary component is set as a second minor axis. In this manner, the major axis and two minor axes orthogonal to the major axis are identified as three axes of the follicle.

Alternatively, in the image data of the follicle, the major axis may be set along a straight line connecting the center of gravity and a pixel which is farthest away from the center of gravity. However, because there may be a case where the farthest pixel is noise or the like, the setting of the major axis by the primary component analysis is more desirable.

When three axes are identified by the three-axes calculating unit 30, a diagnostic coordinate system setting unit 40 sets a diagnostic coordinate system based on the form of the follicle. The diagnostic coordinate system setting unit 40 sets a diagnostic coordinate system having three axes of the follicle as the coordinate axes.

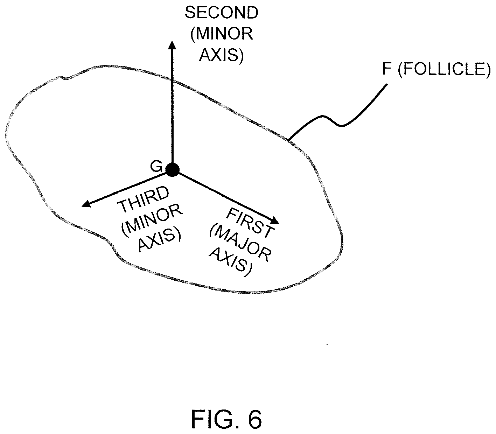

FIG. 6 is a diagram showing the diagnostic coordinate system based on the follicle. The diagnostic coordinate system setting unit 40 sets, as the diagnostic coordinate system, an orthogonal coordinate system shown in FIG. 6 and having, as an origin of the coordinates, a position of the center of gravity G of the follicle F, and having, as the coordinate axes, a first axis in the direction of the first primary component; that is, the direction of the major axis of the follicle F, a second axis in the direction of the second primary component; that is, the direction of one minor axis of the follicle F, and a third axis in the direction of the third primary component; that is, the direction of the other minor axis of the follicle F.

Referring again to FIG. 1, when the diagnostic coordinate system is set by the diagnostic coordinate system setting unit 40, a coordinate system matching unit 50 matches with each other a display coordinate system forming a basis of the display image and the diagnostic coordinate system, to place the image data of the follicle in the display coordinate system.

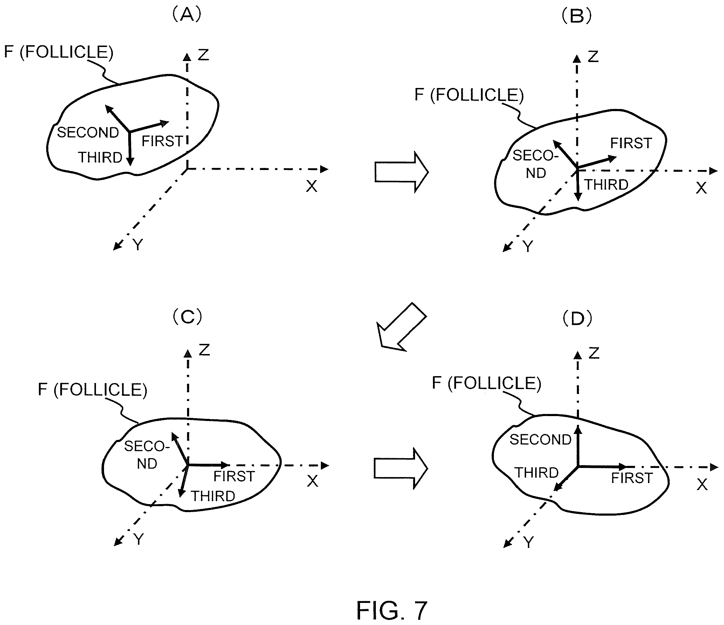

FIG. 7 is a diagram for explaining the matching of the display coordinate system and the diagnostic coordinate system. In FIG. 7, the display coordinate system is shown as an XYZ orthogonal coordinate system. The display coordinate system is a coordinate system which forms a basis when the display image is formed, and is a coordinate system having a clear relative position relationship with respect to the three-dimensional data space (refer to FIG. 3). In the present embodiment, the XYZ orthogonal coordinate system of the three-dimensional data space (refer to FIG. 3) is set as the display coordinate system without further processing.

In FIG. 7, the diagnostic coordinate system is a coordinate system identified by the first axis, second axis, and third axis (refer to FIG. 6). The first through third axes of the diagnostic coordinate system are axes which are obtained by the primary component analysis using, for example, Equations 1 - 3, based on the coordinates of the pixels (voxels) in the three-dimensional data space, and the position and direction in the three-dimensional data space are identified. Therefore, when the XYZ orthogonal coordinate system of the three-dimensional data space is set as the display coordinate system, the position and direction of the diagnostic coordinate system with respect to the display coordinate system are identified.

FIG. 7 shows in (A) an example of the diagnostic coordinate system (first through third axes) with respect to the follicle F identified on the display coordinate system (XYZ axes). Because the diagnostic coordinate system is a coordinate system based on the major axis and the minor axes of the follicle F, the diagnostic coordinate system corresponds to the position and orientation of the follicle F in the display coordinate system.

In consideration of the above, the coordinate system matching unit 50 first translates the diagnostic coordinate system with respect to the display coordinate system to coincide the origin of the display coordinate system and the origin of the diagnostic coordinate system. In this process, the voxel data (image data) related to the follicle F is also translated with the diagnostic coordinate system.

FIG. 7 shows in (B) a state where the diagnostic coordinate system is translated. The origin of the diagnostic coordinate system is moved to the position of the origin of the display coordinate system, and, with this process, the position of the center of gravity of the follicle F which is the origin of the diagnostic coordinate system is moved to the origin of the display coordinate system.

Then, the coordinate system matching unit 50 compares the axis corresponding to the major axis of the follicle F; that is, the first axis of the diagnostic coordinate system, and each of the XYZ axes of the display coordinate system, and identifies, among the XYZ axes, an axis having a smallest angle with respect to the first axis. For example, inner products between the first axis and the XYZ axes are compared to identify the axis having the smallest angle with respect to the first axis. The diagnostic coordinate system is then rotationally moved such that the identified axis and the first axis overlap each other. For example, when the X-axis is identified as the axis having the smallest angle with respect to the first axis, as shown in (C) of FIG. 7, the diagnostic coordinate system is rotated so that the first axis overlaps the X-axis, and the image data of the follicle F are also rotated.

The coordinate systemmatching unit 50 then compares the second axis of the diagnostic coordinate system corresponding to the minor axis of the follicle F and the remaining axes of the display coordinate system, and identifies an axis having a smallest angle with respect to the second axis. For example, when the first axis and the X axis are overlapped, among the remaining axes; that is, the Y-axis and the Z axis, the axis having the smallest angle with respect to the second axis is identified. The diagnostic coordinate system is then rotationally moved so that the identified axis and the second axis overlap each other. For example, when the Z axis is identified as having the smallest angle with respect to the second axis, as shown in (D) of FIG. 7, the diagnostic coordinate system is rotated such that the second axis overlaps the Z axis, and the image data of the follicle F are also rotated.

When the diagnostic coordinate system is an orthogonal coordinate system, if the first axis and the second axis are overlapped with the X axis and the Z axis, the third axis is placed along the Y axis. In (D) of FIG. 7, the third axis and the Y axis are overlapped in the same direction from each other.

Referring again to FIG. 1, when the display coordinate system and the diagnostic coordinate system are matched by the coordinate system matching unit 50 and the image data of the follicle are placed in the display coordinate system, a display image forming unit 60 forms a display image of the follicle based on the image data of the follicle placed in the display coordinate system, and the formed display image is displayed on a display 70. In the formation of the display image, a tomographic image of the follicle in a cross section based on the display coordinate system is formed.

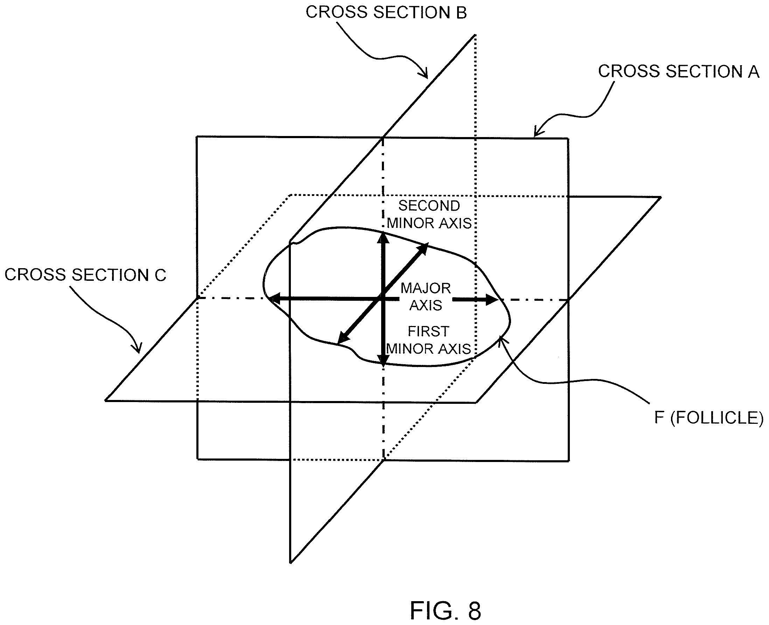

FIG. 8 is a diagram for explaining a cross section based on the display coordinate system. FIG. 8 shows the image data of the follicle F placed in the display coordinate system by the matching of the display coordinate system and the diagnostic coordinate system shown in FIG. 7(D). In FIG. 8, a cross section A is a plane including the Z axis and the X axis of the display coordinate system, a cross section B is a plane including the Y axis and the Z axis of the display coordinate system, and a cross section C is a plane including the X axis and the Y axis of the display coordinate system.

Because the display coordinate system and the diagnostic coordinate system are matched as shown in FIG. 7 (D), the major axis of the follicle F corresponding to the first axis is placed on the X axis, the first minor axis of the follicle F corresponding to the second axis is placed on the Z axis, and the second minor axis of the follicle F corresponding to the third axis is placed on the Y axis. Therefore, in FIG. 8, the cross section A is a cross section including the major axis and the first minor axis of the follicle F, the cross section B is a cross section including the first minor axis and the second minor axis of the follicle F, and the cross section C is a cross section including the major axis and the second minor axis of the follicle F.

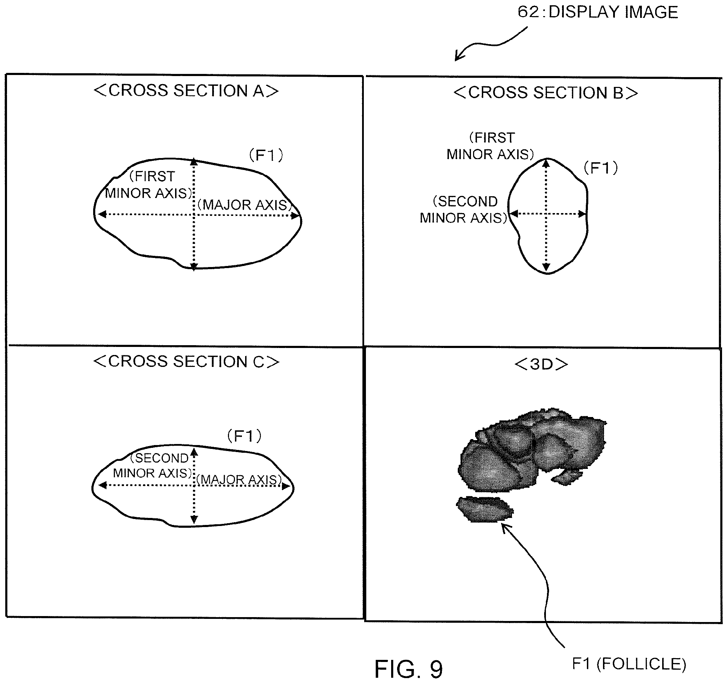

FIG. 9 is a diagram showing a concrete example of a display image 62. Of the images forming the display image 62 shown in FIG. 9, <3D> indicates a three-dimensional image related to a plurality of follicles. The three-dimensional image is formed by, for example, a volume rendering process based on the echo data (voxel data) collected from within the three-dimensional space. By employing a configuration where a position of a viewpoint in the volume rendering process can be changed by the user, it is possible to obtain a three-dimensional image displaying the plurality of follicles from a desired direction.

For example, in the three-dimensional image shown in FIG. 9, when the user displays the plurality of follicles from a desired direction, and moves a display form such as the cursor to position the display form on the position of the desired follicle, the user can select a follicle which the user wishes to diagnose. With this process, a follicle F1 is identified in the three-dimensional image of FIG. 9. Alternatively, in the three-dimensional image, with a display form such as colors and markers or the like, the display may be formed to allow visual distinction of the identified follicle from the other follicles.

For example, when the user identifies the follicle F1 using the three-dimensional image, the three-axes calculating unit 30 identifies the three axes of the follicle F1 (refer to FIG. 5 and Equations 1 - 3), the diagnostic coordinate system setting unit 40 sets the diagnostic coordinate system corresponding to the three axes of the follicle F1 (refer to FIG. 6), and the coordinate system matching unit 50 matches the display coordinate system and the diagnostic coordinate system (refer to FIG. 7). The display image forming unit 60 then forms tomographic images of the follicle F1 at the cross sections A - C (refer to FIG. 8).

In FIG. 9, of the images forming the display image 62, the <cross section A> indicates a tomographic image on the cross section A of the follicle F1, the <cross section B> indicates a tomographic image on the cross section B of the follicle F1, and the <cross section C> indicates a tomographic image on the cross section C of the follicle F1. Because the display coordinate system and the diagnostic coordinate system are matched, on the cross section A, a cross section including the major axis and the first minor axis of the follicle F1 is displayed, on the cross scion B, a cross section including the first minor axis and the second minor axis of the follicle F1 is displayed, and on the cross section C, a cross section including the major axis and the second minor axis of the follicle F1 is displayed.

In this manner, in the present embodiment, the user selects a desired follicle from a plurality of follicles, and a tomographic image including three axes of the identified follicle is formed. Because of this, complicated operation by the user, for example, an operation for setting the cutting plane or the like, can be reduced, and, desirably, the operation for setting the cutting plane can be omitted.

In addition, in the matching of the display coordinate system and the diagnostic coordinate system, the coordinate axes having the minimum intersecting angle are overlapped, and, thus, the rotational movement of the diagnostic coordinate system can be minimized, and visual discomfort of the user felt due to the rotational movement can be minimized.

Alternatively, for the identified follicle F1, measurement values such as the length of the major axis, the lengths of the two minor axes, and the volume may be displayed as a part of the display image 62. In addition, because the plurality of follicles are separated from each other and identified (refer to FIG. 2), the measurement values such as the length of the major axis, the lengths of two minor axes, and the volume for each follicle may be calculated, and a list of the measurement values for the plurality of follicles may be displayed. Moreover, the user may identify a desired follicle from the list of the measurement values, and a cross section of the follicle thus identified may be displayed.

An ultrasound diagnostic apparatus according to a preferred embodiment of the present invention has been described. Alternatively, for example, at least one of the target identifying unit 20, the three-axes calculating unit 30, the diagnostic coordinate system setting unit 40, the coordinate system matching unit 50, and the display image forming unit 60 shown in FIG. 1 may be realized by a computer, and the computer may function as the ultrasound image processor.

[Explanation of Reference Numerals]

10 PROBE; 20 TARGET IDENTIFYING UNIT; 30 THEE-AXES CALCULATING UNIT; 40 DIAGNOSTIC COORDINATE SYSTEM SETTING UNIT; 50 COORDINATE SYSTEM MATCHING UNIT; 60 DISPLAY IMAGE FORMING UNIT; 62 DISPLAY IMAGE 70 DISPLAY

**Claims**

1. An ultrasound diagnostic apparatus comprising:

a probe (10) which is adapted to transmit and receive ultrasound to and from a diagnostic region including a plurality of follicles which are in close contact to each other;
a transmitting and receiving unit (12) which is adapted to control the probe (10) to obtain a reception signal from the diagnostic region;
a target identifying unit (20) which is adapted to identify image data of a diagnosis target in image data of the diagnostic region formed based on the reception signal;
in the image data of the diagnostic region including the plurality of follicles, the target identifying unit (20) is adapted to:

apply an erosion process on the plurality of follicles to separate the plurality of follicles into each individual follicle;

apply a labeling process on the plurality of follicles in the image data to which the erosion process is applied, to assign different labels to the plurality of follicles;

apply a dilation process on each of the plurality of the follicles in the image data to which the labeling process is applied, to restore sizes of the plurality of the follicles while a boundary is formed in an overlap portion between dilation portions which overlap each other due to the dilation process and while assigning the label of each follicle to a dilation portion obtained from the follicle; and

identify, in the image data, the image data of each follicle having the size restored as the image data of the diagnosis target; and a coordinate system setting unit (40) which is adapted to set, based on the image data of the diagnostic target, a diagnostic coordinate system based on a form of the diagnosis target;

a coordinate system matching unit (50) which is adapted to match with each other a display coordinate system forming a basis of a display image and the diagnostic coordinate system, to place the image data of the diagnosis target in the display coordinate system;

a display image forming unit (60) which is adapted to form a display image of the diagnosis target based on the image data of the diagnosis target placed in the display coordinate system, wherein

the coordinate system setting unit (40) is adapted to set the diagnostic coordinate system including, as a coordinate axis, a reference axis identified according to the form of the diagnosis target, and

the coordinate system matching unit (50) is adapted to overlap one coordinate axis of the display coordinate system and the reference axis with each other, to match the display coordinate system and the diagnostic coordinate system with each other.

2. The ultrasound diagnostic apparatus according to Claim 1, wherein

the coordinate system setting unit (40) is adapted to set the diagnostic coordinate system including, as a coordinate axis, a major axis identified along a longitudinal direction of the diagnosis target, and

the coordinate system matching unit (50) is adapted to overlap one coordinate axis of the display coordinate system and the major axis with each other.

3. The ultrasound diagnostic apparatus according to Claim 2, wherein

the coordinate system setting unit (40) is adapted to set the diagnostic coordinate system including, as the coordinate axis and in addition to the major axis of the diagnosis target, a minor axis orthogonal to the major axis, and

the coordinate system matching unit (50) is adapted to overlap one coordinate axis of the display coordinate system and the major axis with each other, and overlaps with each other another coordinate axis of the display coordinate system and the minor axis.

4. The ultrasound diagnostic apparatus according to Claim 2, wherein

the coordinate system matching unit (50) is adapted to overlap with each other a coordinate axis, of the plurality of coordinate axes of the display coordinate system, having a smallest angle with respect to the major axis, and the major axis.

5. The ultrasound diagnostic apparatus according to Claim 3, wherein

the coordinate system matching unit (50) is adapted to overlap with each other a coordinate axis, of the plurality of coordinate axes of the display coordinate system, having a smallest angle with respect to the major axis, and the major axis.

6. The ultrasound diagnostic apparatus according to Claim 1, wherein

the coordinate system setting unit (40) is adapted to set a diagnostic coordinate system obtained by a primary component analysis based on the image data of the diagnosis target.

7. The ultrasound diagnostic apparatus according to Claim 2, wherein

the coordinate system setting unit (40) is adapted to set a diagnostic coordinate system including, as a coordinate axis, a major axis of the diagnosis target obtained by a primary component analysis based on the image data of the diagnosis target.

**8.** The ultrasound diagnostic apparatus according to Claim 3, wherein

the coordinate system setting unit (40) is adapted to set a diagnostic coordinate system including, as coordinate axes, a major axis and a minor axis of the diagnosis target obtained by a primary component analysis based on the image data of the diagnosis target.

**9.** The ultrasound diagnostic apparatus according to Claim 2, wherein

the coordinate system setting unit (40) is adapted to set a diagnostic coordinate system including, as a coordinate axis, a major axis of the diagnosis target obtained by a primary component analysis based on the image data of the diagnosis target, and
the coordinate system matching unit (50) is adapted to overlap with each other a coordinate axis, of the plurality of coordinate axes of the display coordinate system, having a smallest angle with respect to the major axis, and the major axis.

**10.** The ultrasound diagnostic apparatus according to Claim 3, wherein

the coordinate system setting unit (40) is adapted to set a diagnostic coordinate system including, as coordinate axes, a major axis and a minor axis of the diagnosis target obtained by a primary component analysis based on the image data of the diagnosis target, and
the coordinate system matching unit (50) is adapted to overlap with each other a coordinate axis, of the plurality of coordinate axes of the display coordinate system, having a smallest angle with respect to the major axis, and the major axis.

**11.** The ultrasound diagnostic apparatus according to Claim 1, wherein

the target identifying unit (20) is adapted to repeatedly execute an erosion process to stepwise erode the plurality of follicles n times (where n is a natural number), and to repeatedly execute a dilation process to stepwise dilate the plurality of follicles n times.

**Patentansprüche**

**1.** Ultraschalldiagnosegerät, welches aufweist:

eine Sonde (10), welche ausgebildet ist, um Ultraschall zu und von einem Diagnosebereich zu senden und zu empfangen, welcher eine Vielzahl von Follikeln aufweist, welche zueinander in engem Kontakt sind;
eine Sende- und Empfangseinheit (12), welche ausgebildet ist, um die Sonde (10) zu steuern, um ein Empfangssignal von dem Diagnosebereich zu erhalten;
eine Zielerkennungseinheit (20), welche ausgebildet ist, um Bilddaten eines Diagnoseziels in Bilddaten des Diagnosebereichs zu erkennen, welche basierend auf dem Empfangssignal gebildet sind;
wobei die Zielerkennungseinheit (20) ausgebildet ist, um in den Bilddaten des Diagnosebereichs, welcher die Vielzahl von Follikeln aufweist:

einen Erosionsvorgang auf die Vielzahl von Follikeln anzuwenden, um die Vielzahl von Follikeln in jeweils individuelle Follikel zu trennen;
einen Kennzeichnungsvorgang auf die Vielzahl von Follikeln in den Bilddaten anzuwenden, auf welche der Erosionsvorgang angewandt worden ist, um der Vielzahl von Follikeln verschiedene Kennzeichnungen zuzuordnen;
einen Dilatationsvorgang auf jeden der Vielzahl der Follikel in den Bilddaten anzuwenden, auf welche der Kennzeichnungsvorgang angewandt worden ist, um Größen der Vielzahl der Follikel wiederherzustellen, während in einem Überlappungsbereich zwischen Dilatationsbereichen, welche einander wegen des Dilatationsvorgangs überlappen, eine Grenze gebildet wird und während die Kennzeichnung von jedem Follikel einem Dilatationsbereich zugeordnet wird, welcher ausgehend von den Follikeln erhalten wird; und

die Bilddaten von jedem Follikel, welches die wiederhergestellte Größe hat, in den Bilddaten als die Bilddaten des Diagnoseziels zu erkennen; und

eine Koordinatensystembestimmungseinheit (40), welche ausgebildet ist, um, basierend auf den Bilddaten des Diagnoseziels, ein Diagnosekoordinatensystem basierend auf einer Form des Diagnoseziels festzulegen;

eine Koordinatensystemanpassungseinheit (50), welche ausgebildet ist, um ein Anzeigekoordinatensystem, welches eine Basis eines Anzeigebilds bildet, und das Diagnosekoordinatensystem aneinander anzupassen, um die Bilddaten des Diagnoseziels in dem Anzeigekoordinatensystem zu platzieren;

eine Anzeigebildbildungseinheit (60), welche ausgebildet ist, um ein Anzeigebild des Diagnoseziels basierend auf den Bilddaten des Diagnoseziels zu bilden, welche in dem Anzeigekoordinatensystem platziert worden sind, wobei die Koordinatensystembestimmungseinheit (40) ausgebildet ist, um das Diagnosekoordinatensystem festzulegen, welches als eine Koordinatenachse eine Referenzachse aufweist, welche gemäß der Form des Diagnoseziels erkannt worden ist, und

die Koordinatensystemanpassungseinheit (50) ausgebildet ist, um eine Koordinatenachse des Anzeigekoordinatensystems und die Referenzachse miteinander zu überlagern, um das Anzeigekoordinatensystem und das Diagnosekoordinatensystem aneinander anzupassen.

2. Ultraschalldiagnosegerät nach Anspruch 1, wobei
die Koordinatensystembestimmungseinheit (40) ausgebildet ist, um das Diagnosekoordinatensystem festzulegen, welches als eine Koordinatenachse eine Hauptachse aufweist, welche entlang einer longitudinalen Richtung des Diagnoseziels erkannt worden ist, und
die Koordinatensystemanpassungseinheit (50) ausgebildet ist, um eine Koordinatenachse des Anzeigekoordinatensystems und die Hauptachse miteinander zu überlagern.

3. Ultraschalldiagnosegerät nach Anspruch 2, wobei
die Koordinatensystembestimmungseinheit (40) ausgebildet ist, um das Diagnosekoordinatensystem festzulegen, welches als die Koordinatenachse und zusätzlich zu der Hauptachse des Diagnoseziels eine Nebenachse aufweist, welche orthogonal zu der Hauptachse ist, und
die Koordinatensystemanpassungseinheit (50) ausgebildet ist, um eine Koordinatenachse des Anzeigekoordinatensystems und die Hauptachse miteinander zu überlagern, und eine andere Koordinatenachse des Anzeigekoordinatensystems und die Nebenachse miteinander überlagert.

4. Ultraschalldiagnosegerät nach Anspruch 2, wobei
die Koordinatensystemanpassungseinheit (50) ausgebildet ist, um eine Koordinatenachse der Vielzahl von Koordinatenachsen des Anzeigekoordinatensystems, welche einen kleinsten Winkel bezüglich der Hauptachse hat, und die Hauptachse miteinander zu überlagern.

5. Ultraschalldiagnosegerät nach Anspruch 3, wobei
die Koordinatensystemanpassungseinheit (50) ausgebildet ist, um eine Koordinatenachse der Vielzahl von Koordinatenachsen des Anzeigekoordinatensystems, welche einen kleinsten Winkel bezüglich der Hauptachse hat, und die Hauptachse miteinander zu überlagern.

6. Ultraschalldiagnosegerät nach Anspruch 1, wobei
die Koordinatensystembestimmungseinheit (40) ausgebildet ist, um ein Diagnosekoordinatensystem festzulegen, welches durch eine Hauptkomponentenanalyse basierend auf den Bilddaten des Diagnoseziels erhalten wird.

7. Ultraschalldiagnosegerät nach Anspruch 2, wobei
die Koordinatensystembestimmungseinheit (40) ausgebildet ist, um ein Diagnosekoordinatensystem festzulegen, welches als eine Koordinatenachse eine Hauptachse des Diagnoseziels aufweist, welche durch eine Hauptkomponentenanalyse basierend auf den Bilddaten des Diagnoseziels erhalten wird.

8. Ultraschalldiagnosegerät nach Anspruch 3, wobei
die Koordinatensystembestimmungseinheit (40) ausgebildet ist, um ein Diagnosekoordinatensystem festzulegen, welches als Koordinatenachsen eine Hauptachse und eine Nebenachse des Diagnoseziels aufweist, welche durch eine Hauptkomponentenanalyse basierend auf den Bilddaten des Diagnoseziels erhalten werden.

9. Ultraschalldiagnosegerät nach Anspruch 2, wobei
die Koordinatensystembestimmungseinheit (40) ausgebildet ist, um ein Diagnosekoordinatensystem festzulegen, welches als eine Koordinatenachse eine Hauptachse des Diagnoseziels aufweist, welche durch eine Hauptkomponentenanalyse basierend auf den Bilddaten des Diagnoseziels erhalten wird, und
die Koordinatensystemanpassungseinheit (50) ausgebildet ist, um eine Koordinatenachse der Vielzahl von Koordinatenachsen des Anzeigekoordinatensystems, welche einen kleinsten Winkel bezüglich der Hauptachse hat, und die Hauptachse miteinander zu überlagern.

10. Ultraschalldiagnosegerät nach Anspruch 3, wobei
die Koordinatensystembestimmungseinheit (40) ausgebildet ist, um ein Diagnosekoordinatensystem festzulegen, welches als Koordinatenachsen eine Hauptachse und eine Nebenachse des Diagnoseziels aufweist, welche durch eine Hauptkomponentenanalyse basierend auf den Bilddaten des Diagnoseziels erhalten werden, und
die Koordinatensystemanpassungseinheit (50) ausgebildet ist, um eine Koordinatenachse der Vielzahl von Koordinatenachsen des Anzeigekoordinatensystems, welche einen kleinsten Winkel bezüglich der Hauptachse hat, und die Hauptachse miteinander zu überlagern.

11. Ultraschalldiagnosegerät nach Anspruch 1, wobei
die Zielerkennungseinheit (20) ausgebildet ist, wiederholt einen Erosionsvorgang auszuführen, um schrittweise die Vielzahl von Follikeln n Male (wobei n eine natürliche Zahl ist) zu erodieren und um einen Dilatationsvorgang wiederholt auszuführen, um die Vielzahl von Follikeln n Male wiederholt zu dilatieren.

## Revendications

1. Appareil de diagnostic à ultrasons comprenant :

une sonde (10) qui est destinée à transmettre et à recevoir des ultrasons vers et depuis une région de diagnostic incluant une pluralité de follicules qui sont en contact étroit les uns avec les autres ;
une unité de transmission et de réception (12) qui est destinée à commander la sonde (10) pour obtenir un signal de réception provenant de la région de diagnostic ;
une unité d'identification de cible (20) qui est destinée à identifier des données d'images d'une cible de diagnostic dans des données d'images de la région de diagnostic formées à partir du signal de réception ;
dans les données d'images de la région de diagnostic incluant la pluralité de follicules, une unité d'identification de cible (20) est destinée à :

appliquer un processus d'érosion sur la pluralité de follicules pour séparer la pluralité de follicules en chaque follicule individuel ;
appliquer un processus d'étiquetage sur la pluralité de follicules dans les données d'images auxquels le processus d'érosion est appliqué, pour attribuer différentes étiquettes à la pluralité de follicules ;
appliquer un processus de dilatation sur chaque follicule de la pluralité de follicules dans les données d'images auxquels le processus d'étiquetage est appliqué, pour rétablir des dimensions de la pluralité de follicules alors qu'une limite est formée dans une partie de chevauchement entre des parties de dilatation qui se chevauchent les unes avec les autres en raison du processus du dilatation et tout en attribuant une étiquette de chaque follicule à une partie de dilatation obtenue à partir du follicule ; et
identifier, dans les données d'images, les données d'images de chaque follicule ayant la taille rétablie comme les données d'images de la cible de diagnostic ; et une unité de paramétrage de système de coordonnées (40) qui est destinée à paramétrer, sur la base des données d'images de la cible de diagnostic, un système de coordonnées de diagnostic basé sur une forme de la cible de diagnostic ;
une unité de mise en correspondance de systèmes de coordonnées (50) qui est destinée à mettre en correspondance l'un avec l'autre un système de coordonnées d'affichage constituant une base d'une image d'affichage et le système de coordonnées de diagnostic, pour placer les données d'images de la cible de diagnostic dans le système de coordonnées d'affichage ;
une unité de formation d'image d'affichage (60) qui est destinée à former une image d'affichage de la cible de diagnostic sur la base des données d'images de la cible de diagnostic placées dans le système de coordonnées d'affichage, dans lequel
l'unité de paramétrage de système de coordonnées (40) est destinée à paramétrer le système de coordonnées de diagnostic incluant, comme axe de coordonnées, un axe de référence identifié selon la forme de la cible de diagnostic, et
l'unité de mise en correspondance de systèmes de coordonnées (50) est destinée à faire se chevaucher un premier axe de coordonnées du système de coordonnées d'affichage et l'axe de référence l'un avec l'autre, pour mettre en correspondance le système de coordonnées d'affichage et le système de coordonnées de diagnostic l'un avec l'autre.

2. Appareil de diagnostic à ultrasons selon la revendication 1, dans lequel
l'unité de paramétrage de système de coordonnées (40) est destinée à paramétrer le système de coordonnées de diagnostic incluant, comme axe de coordonnées, un axe principal identifié le long d'une direction longitudinale de la cible de diagnostic, et
l'unité de mise en correspondance de systèmes de coordonnées (50) est destinée à faire se chevaucher un axe de coordonnées du système de coordonnées d'affichage et l'axe principal l'un avec l'autre.

3. Appareil de diagnostic à ultrasons selon la revendication 2, dans lequel
l'unité de paramétrage de système de coordonnées (40) est destinée à paramétrer le système de coordonnées de diagnostic incluant, comme axe de coordonnées et en plus de l'axe principal de la cible de diagnostic, un axe secondaire orthogonal à l'axe principal, et
l'unité de mise en correspondance de systèmes de coordonnées (50) est destinée à faire se chevaucher un axe de coordonnées du système de coordonnées d'affichage et l'axe principal l'un avec l'autre, et fait se chevaucher l'un avec l'autre un autre axe de coordonnées du système de coordonnées d'affichage et l'axe secondaire.

4. Appareil de diagnostic à ultrasons selon la revendication 2, dans lequel
l'unité de mise en correspondance de systèmes de coordonnées (50) est destinée à faire se chevaucher l'un avec l'autre un axe de coordonnées, de la pluralité d'axes de coordonnées du système de coordonnées d'affichage, ayant un angle le plus petit par rapport à l'axe principal, et l'axe principal.

5. Appareil de diagnostic à ultrasons selon la revendication 3, dans lequel
l'unité de mise en correspondance de systèmes de coordonnées (50) est destinée à faire se chevaucher l'un avec l'autre un axe de coordonnées, de la pluralité d'axes de coordonnées du système de coordonnées d'affichage, ayant un angle le plus petit par rapport à l'axe principal, et l'axe principal.

6. Appareil de diagnostic à ultrasons selon la revendication 1, dans lequel
l'unité de paramétrage de système de coordonnées (40) est destinée à paramétrer un système de coordonnées de diagnostic obtenu par une analyse en composantes principales basée sur les données d'images de la cible de diagnostic.

7. Appareil de diagnostic à ultrasons selon la revendication 2, dans lequel
l'unité de paramétrage de système de coordonnées (40) est destinée à paramétrer un système de coordonnées de diagnostic incluant, comme axe de coordonnées, un axe principal de la cible de diagnostic obtenu par une analyse en composantes principales basée sur les données d'images de la cible de diagnostic.

8. Appareil de diagnostic à ultrasons selon la revendication 3, dans lequel
l'unité de paramétrage de système de coordonnées (40) est destinée à paramétrer un système de coordonnées de diagnostic incluant, comme axes de coordonnées, un axe principal et un axe secondaire de la cible de diagnostic obtenus par une analyse en composantes principales basée sur les données d'images de la cible de diagnostic.

9. Appareil de diagnostic à ultrasons selon la revendication 2, dans lequel
l'unité de paramétrage de système de coordonnées (40) est destinée à paramétrer un système de coordonnées de diagnostic incluant, comme axe de coordonnées, un axe principal de la cible de diagnostic obtenu par une analyse en composantes principales basée sur les données d'images de la cible de diagnostic, et
l'unité de mise en correspondance de systèmes de coordonnées (50) est destinée à faire se chevaucher l'un avec l'autre un axe de coordonnées, de la pluralité d'axes de coordonnées du système de coordonnées d'affichage, ayant un angle le plus petit par rapport à l'axe principal, et l'axe principal.

10. Appareil de diagnostic à ultrasons selon la revendication 3, dans lequel
l'unité de paramétrage de système de coordonnées (40) est destinée à paramétrer un système de coordonnées de diagnostic incluant, comme axes de coordonnées, un axe principal et un axe secondaire de la cible de diagnostic obtenus par une analyse en composantes principales basée sur les données d'images de la cible de diagnostic, et
l'unité de mise en correspondance de systèmes de coordonnées (50) est destinée à faire se chevaucher l'un avec l'autre un axe de coordonnées, de la pluralité d'axes de coordonnées du système de coordonnées d'affichage, ayant un angle le plus petit par rapport à l'axe principal, et l'axe principal.

**11.** Appareil de diagnostic à ultrasons selon la revendication 1, dans lequel l'unité d'identification de cible (20) est destinée à exécuter de manière répétée un processus d'érosion pour éroder pas à pas la pluralité de follicules n fois (où n est un nombre entier), et à exécuter de manière répétée un processus de dilatation pour dilater pas à pas la pluralité de follicules n fois.

FIG. 1

(A) BINARIZATION PROCESS

(B) EROSION AND
SEPARATION PROCESS

(F: FOLLICLE)

(F1)

(F2)    (F3)

(C) LABELING PROCESS

(D) DILATION AND
BOUNDARY PROCESS

LABEL 0

LABEL 1

LABEL 2    LABEL 3

LABEL 0

LABEL 1

LABEL 2    LABEL 3

FIG. 2

EP 2 698 114 B1

100: THREE-DIMENSIONAL
DATA SPACE

120: FILTER (EROSION
OR DILATION)

FIG. 3

<CONDITION TABLE FOR DILATION AND BOUNDARY PROCESS>

| VOXEL OF INTEREST (VOXEL AT CENTER) | PERIPHERAL VOXELS (26 SURROUNDING VOXELS) | OUTPUT VOXEL (VOXEL OF INTEREST AFTER PROCESS) |
|---|---|---|
| LABEL 0 | LABEL 0 FOR ALL VOXELS | LABEL 0 |
| LABEL 0 | THERE EXIST LABELS OTHER THAN LABEL 0 AND THESE LABELS ARE THE SAME LABEL N | LABEL N |
| LABEL 0 | THERE EXIST LABELS OTHER THAN LABEL 0 AND THESE LABELS HAVE DIFFERENT LABEL NUMBERS | LABEL 0 (BOUNDARY) |
| LABEL M (M ≠ 0) | (NO CONDITION) | LABEL M |

FIG. 4

EP 2 698 114 B1

FIG. 5

SECOND
(MINOR
AXIS)

F (FOLLICLE)

G

THIRD
(MINOR
AXIS)

FIRST
(MAJOR
AXIS)

FIG. 6

FIG. 7

FIG. 8

EP 2 698 114 B1

62:DISPLAY IMAGE

| <CROSS SECTION A> | <CROSS SECTION B> |
|---|---|
| (F1)<br>(FIRST MINOR AXIS)<br>(MAJOR AXIS) | (FIRST MINOR AXIS)<br>(F1)<br>(SECOND MINOR AXIS) |
| <CROSS SECTION C> | <3D> |
| (F1)<br>(SECOND MINOR AXIS)<br>(MAJOR AXIS) | F1 (FOLLICLE) |

FIG. 9

EP 2 698 114 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 3802508 B
- US 20090082668 A1
- US 20060280351 A1
- US 20080267499 A1
- US 20100195881 A1
- US 20060285734 A1